# EUROPEAN PATENT SPECIFICATION

(11) **EP 1 180 094 B1**
(45) Date of publication and mention of the grant of the patent: **28.07.2004**
(21) Application number: 00929701.1
(22) Date of filing: 12.05.2000
(51) Int. Cl.: C07C 229/34, A61K 31/195, A61P 25/24, A61P 25/28, A61P 25/02, A61P 25/08

(54) **FUSED POLYCYCLIC AMINO ACIDS AS PHARMACEUTICAL AGENTS**
AMINOSÄUREN MIT POLYCYCLISCHER STRUKTUR ALS PHARMAKA
ACIDES AMINES POLYCYCLIQUES ACCOLES UTILISES COMME AGENTS PHARMACEUTIQUES

(30) Priority: 26.05.1999 US 136100 P
(43) Date of publication of application: 20.02.2002
(73) Proprietor: WARNER-LAMBERT COMPANY, Morris Plains, New Jersey 07950 (US)
(72) Inventor: BRYANS, Justin Stephen, Balsham CB1 6DZ (GB); HORWELL, David Christopher, Cambridge CB2 6SZ (GB); OSBORNE, Simon, Stetchworth-Newmarket, Suffolk CB8 9TZ (GB)
(74) Representative: Stevens, Ian Edward
(86) International application number: PCT/GB2000/001819
(87) International publication number: WO 2000/073259

(56) References cited:
- WO-A-93/08799

## Description

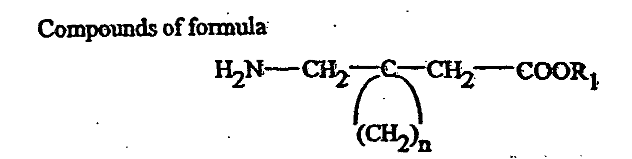

wherein R₁ is hydrogen or a lower alkyl radical and n is 4, 5, or 6 are known in United States Patent Number 4,024,175 and its divisional United States Patent Number 4,087,544. The uses disclosed are: protective effect against cramp induced by thiosemicarbazide; protective action against cardiazole cramp; the cerebral diseases, epilepsy, faintness attacks, hypokinesia, and cranial traumas; and improvement in cerebral functions. The compounds are useful in geriatric patients. 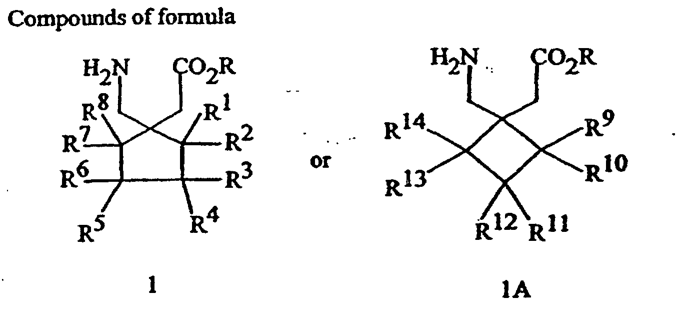 or a pharmaceutically acceptable salt thereof wherein:
- R: is hydrogen or a lower alkyl;
- R¹ to R¹⁴: are each independently selected from hydrogen, straight or branched alkyl of from 1 to 6 carbons, phenyl, benzyl fluorine, chlorine, bromine, hydroxy, hydroxymethyl, amino, aminomethyl, trifluoromethyl, -CO₂H, -CO₂R¹⁵, -CH₂CO₂H, -CH₂CO₂R¹⁵, -OR¹⁵ wherein R¹⁵ is a straight or branched alkyl of from 1 to 6 carbons, phenyl, or benzyl, and R¹ to R⁸ are not simultaneously hydrogen are known in United States Provisional Application Serial Number 60/097,685 filed August 8, 1998.

WO 93/08799 covers a compound of Formula I 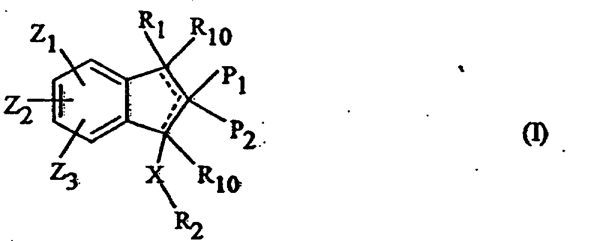 wherein:
- R₁: is -X(CH₂)ₙAr or -X(CH₂)ₙR₈ or 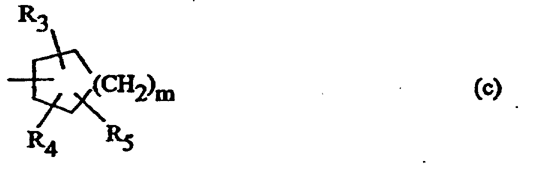
- R₂: is hydrogen, Ar or (c);
- P₁: is -X(CH₂)ₙR₈;
- P₂: is -X(CH₂)ₙR₈, or -XR₉Y;
- R₃ and R₅: are independently hydrogen, R₁₁, OH, C₁₋₈alkoxy, S(O)_{q}R₁₁, N(R₆)₂, Br, F, I, Cl, CF₃, NHCOR₆, -XR₉-Y or -X(CH₂)ₙR₈ wherein the methylene groups of -X(CH₂)ₙR₈ may be unsubstituted or substituted by one or more -(CH₂)ₙAr groups;
- R₄: is hydrogen, R₁₁, OH, C₁₋₅alkoxy, S(O)_{q}R₁₁, N(R₆)₂, -X(R₁₁), Br, F, I, Cl or NHCOR₆ wherein the C₁₋₅alkoxy may be unsubstituted or substituted by OH methoxy or halogen;
- R₆: is independently hydrogen or C₁₋₄alkyl;
- R₇: is independently hydrogen, C₁₋₆alkyl or (CH₂)ₙAr;
- R₈: is hydrogen, R₁₁, CO₂H, PO₃H₂, P(O)(OH) R₇ or tetrazole;
- R₉: is C₁₋₁₀alkyl, C₂₋₁₀alkenyl or phenyl all of which may be unsubstituted or substituted by one or more OH, N(R₆)₂, COOH, halogen or XC₁₋₅alkyl;
- R₁₀: is R₃ or R₄;
- R₁₁: is C₁₋₈alkyl, C₂₋₈alkenyl, C₂₋₈alkynyl all of which may be unsubstituted or substituted by one or more OH, CH₂OH, N(R₆)₂ or halogen;
- X: is (CH₂)ₙ, O, NR₆ or S(O)q;
- Y: is CH₃ or -CH₂X(CH₂)ₙAr;
- Ar: is: 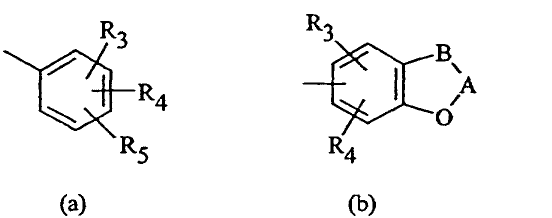 naphthyl, indolyl, pyridyl or thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
- A: is C=O, or [C(R₆)₂]ₘ;
- B: is -CH₂- or -O-;
- Z₁ and Z₂: are independently hydrogen, C₁₋₈alkyl, C₂₋₈alkenyl, C₂₋₈alkynyl, OH, C₁₋₈alkoxy, S(O)_{q}C₁₋₈alkyl, N(R₆)₂, Br, F, I, Cl, NHCOR₆, - X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂, or halogen; or Z₁ and Z₂ together may be -O-A-O- on contiguous carbons;
- Z₃: is Z₁ or XR₉Y;
- q: is zero, one or two;
- n: is an integer from 0 to six;
- m: is 1, 2 or 3; and the dotted line indicates the optional presence of a double bond; or a pharmaceutically acceptable salt thereof; provided that:
- R₂ is not hydrogen when X is S(O)_{q};
- when the optional double bond is present there is only one R₁₀ and there is no P₁;
- the compound of Formula I is not (1RS)-1,3-diphenylindene-2-carboxylic acid; (cis,cis)-(1RS,3SR)-1,3-diphenylindane-2-carboxylic acid; (1RS)-3-[3-Methyl-1-phenyl-(1H)-ind-2-en-1-yl]propionic acid; or (1RS)-2[1,3-diphenyl-(1H)-ind-2-en-2-yl)]ethanoic acid.

WO 94/25013 covers a compound of Formula (I): 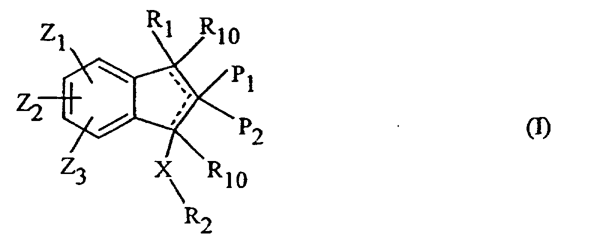 wherein:
- R₁: is -X(CH₂)ₙAr or -X(CH₂)ₙR₈ or 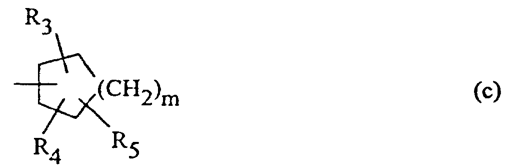
- R₂: is hydrogen, Ar, C₁₋₄alkyl or (c);
- P₁: is -X(CH₂)ₙR₈;
- P₂: is -X(CH₂)ₙR₈, or -X-R₉-Y;
- R₃ and R₅: are independently hydrogen, R₁₁, OH, C₁₋₈alkoxy, S(O)_{q}R₁₁, N(R₆)₂, Br, F, I, Cl, CF₃, NHCOR₆, R₁₁CO₂R₇, -X-R₉-Y, or -X(CH₂)ₙR₈ wherein each methylene group within -X(CH₂)ₙR₈ may be unsubstituted or substituted by one or two -(CH₂)ₙAr groups;
- R₄: is hydrogen, R₁₁, OH, C₁₋₅alkoxy, S(O)_{q}R₁₁, N(R₆)₂, -X(R₁₁), Br, F, I, Cl, or NHCOR₆ wherein the C₁₋₅alkoxy may be unsubstituted or substituted by OH, methoxy or halogen;
- R₆: is independently hydrogen or C₁₋₄alkyl;
- R₇: is independently hydrogen, C₁₋₁₀alkyl, C₂₋₁₀alkenyl or C₂₋₈alkenyl, all of which may be unsubstituted or substituted by one or more OH, N(R₆)₂, CO₂R₁₂, halogen or XC₁₋₅alkyl; or R₇ is (CH₂)ₙAr;
- R₈: is hydrogen, R₁₁, CO₂R₇, CO₂C(R11)₂O(CO)XR₇, PO₃(R₇)₂, SO₂NR₇R₁₁, CONR₇SO₂R₁₁, SO₃R₇, SO₂R₇, P(O)(OR₇)R₇, CN, C(O)N(R₆)₂, - CO₂(CH₂)ₘC(O)N(R₆)₂, C(R₁₁)₂N(R₇)₂, tetrazole or OR₆;
- R₉: is (CH₂)ₙ, divalent C₁₋₁₀alkyl, divalent C₂₋₁₀alkenyl or phenyl, all of which may be unsubstituted or substituted by one or more OH, N(R₆)₂, COOH, halogen, or may be >C=O or XC₁₋₅alkyl;
- R₁₀: is R₃ or R₄;
- R₁₁: is hydrogen, Ar, C₁₋₈alkyl, C₂₋₈alkenyl, C₂₋₈alkynyl, all of which may be unsubstituted or substituted by one or more OH, CH₂OH, N(R₆)₂ or halogen:
- R₁₂: is hydrogen, C₁₋₆alkyl, C₂₋₆alkenyl or C₂₋₇alkynyl;
- X: is (CH₂)ₙ, O, NR₆ or S(O)_{q};
- Y: is CH₃ or X(CH₂)ₙAr;
- Ar: is: 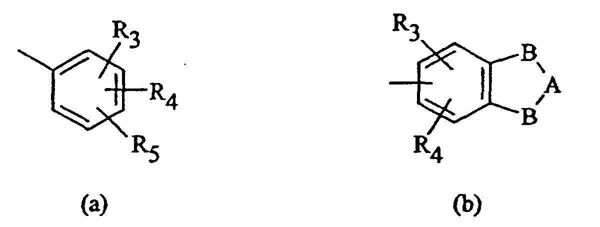 naphthyl, indolyl, pyridyl, thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
- A: is C=O, or [C(R₆)₂]ₘ;
- B: is -CH₂- or -O-;
- Z₁ and Z₂: are independently hydrogen, C₁₋₈alkyl, C₂₋₈alkenyl, C₂₋₈alkynyl, OH, C₁₋₈alkoxy, S(O)qC₁₋₈alkyl, N(R₆)₂, Br, F, I, Cl, NHCOR₆, -X-R₉-Y, -X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂, or halogen; or Z₁ and Z₂ together may be -O-A-O- on contiguous carbons;
- Z₃: is Z₁ or ―X-R₉-Y;
- q: is zero, one or two;
- n: is an integer from 0 to six;
- m: is 1, 2 or 3;
and the dotted line indicates the optional presence of a double bond; or a pharmaceutically acceptable salt thereof: provided that:
- R₂: is hydrogen, Ar or (c);
- P₁: is -X(CH₂)ₙR₈;
- P₂: is -X(CH₂)ₙR₈, or -XR₉Y;
- R₃ and R₅: are independently hydrogen, R₁₁, OH, C₁₋₈alkoxy, S(O)_{q}R₁₁, N(R₆)₂, Br, F, I, Cl, CF₃, NHCOR₆, -XR₉-Y or -X(CH₂)ₙR₈ wherein the methylene groups of -X(CH₂)ₙR₈ may be unsubstituted or substituted by one or more -(CH₂)ₙAr groups:
- R₄: is hydrogen, R₁₁, OH, C₁₋₅alkoxy, S(O)_{q}R₁₁, N(R₆)₂, -X(R₁₁), Br, F, I, Cl or NHCOR₆ wherein the C₁₋₅alkoxy may be unsubstituted or substituted by OH, methoxy or halogen;
- R₆: is independently hydrogen or C₁₋₄alkyl;
- R₇: is independently hydrogen, C₁₋₆alkyl or (CH₂)ₙAr;
- R₈: is hydrogen, R₁₁, CO₂H, PO₃H₂, P(O)(OH)R₇ or tetrazole;
- R₉: is C₁₋₁₀alkyl, C₂₋₁₀alkenyl or phenyl all of which may be unsubstituted or substituted by one or more OH, N(R₆)₂, COOH, halogen or XC₁₋₅alkyl;
- R₁₀: is R₃ or R₄;
- R₁₁: is C₁₋₈alkyl, C₂₋₈alkenyl, C₂₋₈alkynyl all of which may be unsubstituted or substituted by one or more OH, CH₂OH, N(R₆)₂ or halogen;
- X: is (CH₂)ₙ, O, NR₆ or S(O)_{q};
- Y: is CH₃ or -CH₂X(CH₂)ₙAr;
- Ar: is: 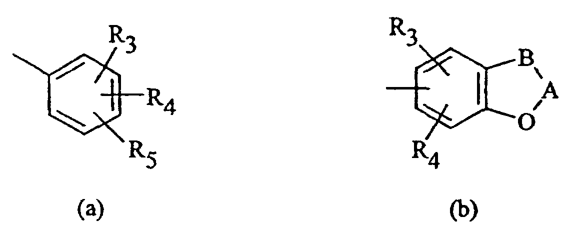 naphthyl, indolyl, pyridyl or thienyl, oxazolidinyl, oxazolyl, thiazolyl, isothiazolyl, pyrazolyl, triazolyl, tetrazolyl, imidazolyl, imidazolidinyl, thiazolidinyl, isoxazolyl, oxadiazolyl, thiadiazolyl, morpholinyl, piperidinyl, piperazinyl, pyrrolyl, or pyrimidyl; all of which may be unsubstituted or substituted by one or more R₃ or R₄ groups;
- A: is C=O, or [C(R₆)₂]ₘ;
- B: is -CH₂- or -O-;
- Z₁ and Z₂: are independently hydrogen, C₁₋₈alkyl, C₂₋₈alkenyl, C₂₋₈alkynyl, OH, C₁₋₈alkoxy, S(O)qC₁₋₈alkyl, N(R₆)₂, Br, F, I, Cl, NHCOR₆, - X(CH₂)ₙR₈, phenyl, benzyl or C₃₋₆cycloalkyl wherein the C₁₋₈alkyl, C₂₋₈alkenyl or C₂₋₈alkynyl may be optionally substituted by COOH, OH, CO(CH₂)ₙCH₃, CO(CH₂)ₙCH₂N(R₆)₂, or halogen; or Z₁ and Z₂ together may be -O-A-O- on contiguous carbons;
- Z₃: is Z₁ or XR₉Y;
- q: is zero, one or two;
- n: is an integer from 0 to six;
- m: is 1, 2, or 3; are excluded.
CO(CH₂)ₙCH₃, CO(CH₂)ₙCH₂N(R₆)2, or halogen; or Z₁ and Z₂ together may be -O-A-O- on contiguous carbons;
- Z₃: is Z₁ or -X-R₉-Y;
- q: is zero, one or two;
- n: is an integer from 0 to six;
- m: is 1, 2, or 3; and the dotted line indicates the optional presence of a double bond; or a pharmaceutically acceptable salt thereof; provided that
- R₂ is not hydrogen when X is S(O)_{q};
- when the optional double bond is present there is only one R₁₀ and there is no P₁ and P₂ is not NR₆R₉Y;
- when the optional double bond is present in Formula (I) and X-R₂ is attached to the double bond, X is not NR₆;
- when the optional double bond is present and R₁ is attached drirectly to the double bond, R₁ is not NR₆AR;
- when R₃, R₅, Z₁, Z₂, or Z₃ is X(CH₂)ₙR₈ and n is not 0, X is oxygen or NR₆ when R₈ is OR₆ or CO₂H;
- when R₈ is CO₂C(R₁₁)₂O(CO)XR₇, X is not S(O)_{q};
- the compound of Formula I is not (1RS)-1,3-diphenylindene-2-carboxylic acid; (cis,cis)-(1RS,3SR)-1,3-diphenylindane-2-carboxylic acid; (1RS)-3-[3-Methyl-1-phenyl-(1H)-ind-2-en-1-yl]propionic acid; or (1RS)-2[1,3-diphenyl-(1H)-ind-2-en-2-2-yl]ethanoic acid; 1,3-diphenyl;-1-ethoxyindene-2-carboxylic acid; 1,2,3-triphenylindene; 1,3-diphenylindene; 1-(2,3-dimethyl-2-buten-yl)-1,3-diphenylindene; 1,3-diphenyl-2-methylindene; 1-3-diphenyl-2-methylindane; 1,3-diphenylindane; 5,6-dimethoxy-1,3-dimethoxyindene; 1,3-bis(4,5-dimethoxy-2-hydroxyphenyl)-5,6-dimethoxyindane; 1,3-bis(3,4-dimethoxyphenyl)-5,6-dimethoxyindane; 1,3-diphenyl-2-methoxyidene, 1,3-diphenyl-2-ethoxyindene, or 5-fluoro-2-methyl-indene-3-acetic acid;
and further provided that compounds wherein:
- R₁: is -X(CH₂)ₙAr or -X(CH₂)ₙR₈ or

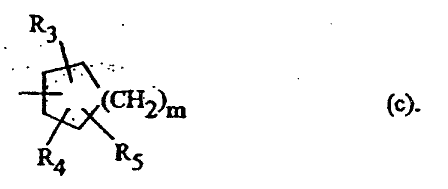

### SUMMARY OF THE INVENTION

The compounds, prodrugs, and pharmaceutically acceptable salts are useful in a variety of disorders which include: epilepsy, faintness attacks, hypokinesia, cranial disorders, neurodegenerative disorders, depression, anxiety, panic, pain, neuropathological and sleep disorders.

The compounds are those of Formula I 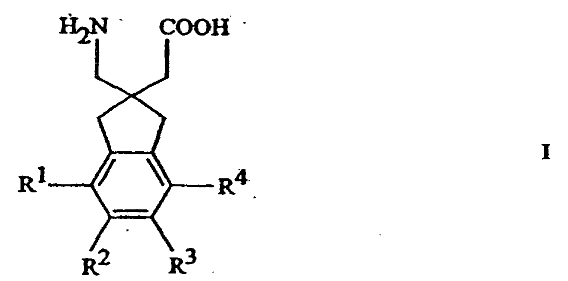 wherein R¹ to R⁴ are as described below.

Preferred compounds of the invention are those wherein R¹ to R⁴ are absent.

Especially preferred are:
(2-aminomethyl-indan-2-yl)-acetic acid;
(2-aminomethyl-4,7-dimethyl-indan-2-yl)-acetic acid;
(2-aminomethyl-5,6-dimethyl-indan-2-yl)-acetic acid;
(2-aminomethyl-4-methyl-indan-2-yl)-acetic acid;
(2-aminomethyl-5-methyl-indan-2-yl)-acetic acid;
(4,7-dichloro-2-aminomethyl-indan-2-yl)-acetic acid;
(5,6-dichloro-2-aminomethyl-indan-2-yl)-acetic acid;
(4-chloro-2-aminomethyl-indan-2-yl)-acetic acid;
(5-chloro-2-aminomethyl-indan-2-yl)-acetic acid;
(2-aminomethyl-2,3-dihydro-1H-cyctopenta[b]naphthalen-2-yl)-acetic acid;
(2-Aminomethyl-2,3-dihydro-1H-cyclopenta[a]naphthalen-2-yl)-acetic acid;
(2-Aminomethyl-4,7-dimethoxy-indan-2-yl)-acetic acid;
(2-Aminomethyl-5,6-dimethoxy-indan-2-yl)-acetic acid;
(2-Aminomethyl-4-methoxy-indan-2-yl)-acetic acid;
(2-Aminomethyl-5-methoxy-indan-2-yl)-acetic acid;
(2-Aminomethyl-4,7-dibromo-indan-2-yl)-acetic acid;
(2-Aminomethyl-5,6-dibromo-indan-2-yl)-acetic acid;
(2-Aminomethyl-4-bromo-indan-2-yl)-acetic acid;
(2-Aminomethyl-5-bromo-indan-2-yl)-acetic acid;
(4-Amino-2-aminomethyl-indan-2-yl)-acetic acid;
(5-Amino-2-aminomethyl-indan-2-yl)-acetic acid;
2-Aminomethyl-2-carboxymethyl-indan-4-carboxylic acid;
2-Aminomethyl-2-carboxymethyl-indan-4-carboxylic acid;
2-Aminomethyl-2-carboxymethyl-indan-5-carboxylic acid;
2-Aminomethyl 2-carboxymethyl-indan-4-carboxylic acid methyl ester;
2-Aminomethyl-2-carboxymethyl-indan-5-carboxylic acid methyl ester;
(2-Aminomethyl-4-methylamino-indan-2-yl)-acetic acid;
(2-Aminomethyl-5-methylamino-indan-2-yl)-acetic acid;
(2-Aminomethyl-4-cyano-indan-2-yl)-acetic acid;
(2-Aminomethyl-5-cyano-indan-2-yl)-acetic acid;
(2-Aminomethyl-4-trifluoromethyl-indan-2-yl)-acetic acid;
(2-Aminomethyl-4,7-bis-trifluoromethyl-indan-2-yl)-acetic acid;
(2-Aminomethyl-5-trifluoromethyl-indan-2-yl)acetic acid;
(2-Aminomethyl-5,6-bis-trifluoromethyl-indan-2-yl)-acetic acid;
(2-Aminomethyl-4-hydroxy-indan-2-yl)-acetic acid;
(2-Aminomethyl-5-hydroxy-indan-2-yl)-acetic acid;
(2-Aminomethyl-5-phenyl-indan-2-yl)-acetic acid;
(2-Aminomethyl-4-phenyl-indan-2-yl)-acetic acid;
(2-Aminomethyl-5,6-diphenyl-indan-2-yl)-acetic acid;
(2-Aminomethyl-4,7-diphenyl-indan-2-yl)-acetic acid;
(2-Aminomethyl-4,5-diphenyl-indan-2-yl)-acetic acid;
(2-Aminomethyl-4-isopropyl-indan-2-yl)-acetic acid;
(2-Aminomethyl-5-isopropyl-indan-2-yl)-acetic acid;
(2-Aminomethyl-4,7-diisopropyl-indan-2-yl)-acetic acid;
(2-Aminomethyl-4-isobutyl-indan-2-yl)-acetic acid;
(2-Aminomethyl-5-isobutyl-indan-2-yl)-acetic acid;
(2-Aminomethyl-4-dimethylamino-indan-2-yl)-acetic acid;
(2-Aminomethyl-5-dimethylamino-indan-2-yl)-acetic acid;
(2-Aminomethyl-4-iodo-indan-2-yl)-acetic acid;
(2-Aminomethyl-5-iodo-indan-2-yl)-acetic acid;
(2-Aminomethyl-4-nitro-indan-2-yl)-acetic acid; and
(2-Aminomethyl-5-nitro-indan-2-yl)-acetic acid.

The invention further is pharmaceutical compositions of one or more compounds of Formula I above in combination with a pharmaceutically acceptable carrier.

The compounds of the invention are agents useful in the treatment of epilepsy, faintness attacks, hypokinesia, cranial disorders, neurodegenerative disorders, depression, anxiety, panic, pain, neuropathological disorders, and sleep disorders.

### DETAILED DESCRIPTION OF THE INVENTION

The compounds of Formula I below will be more lipophilic in nature and therefore more able to pass into the gut and across the blood/brain barrier by passive diffusion. The compounds are expected to have a longer duration of action.

The compounds of the instant invention and their pharmaceutically acceptable salts and prodrugs are as defined by Formula I above or a pharmaceutically acceptable salt thereof wherein:
- R¹, R², R³, and R⁴: are each independently absent or selected from
hydrogen,
halogen,
-CN,
-NO₂,
-CF₃,
-OH,
-SH,
-Z-A wherein Z is -O-, -S-, or (CH₂)ₙ wherein n is an integer of from 0 to 6 and A is a straight, branched, or cyclic alkyl of from 1 to 6 carbons or phenyl substituted with from 1 to 5 substituents selected from H, F, Cl, Br, I, -CN, -NO₂, -CF₃, -OCH₃, or a straight, branched, or cyclic alkyl of from 1 to 6 carbons; 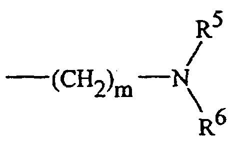 wherein m is an integer of from 0 to 6, R⁵ is H, a straight or branched or cyclic alkyl of from 1 to 6 carbons or phenyl substituted with from 1 to 5 groups each independently selected from H, F, Cl, Br, I, -CN, -NO₂, and -CF₃, and R⁶ is H, straight or branched or cyclic alkyl of from 1 to 6 carbons; 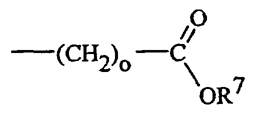 wherein o is an integer of from 0 to 6, and R⁷ is H, straight chain, branched, or cyclic alkyl of from 1 to 6 carbons, or phenyl substituted with from 1 to 5 groups each independently selected from H, F, Cl, Br, I, -CN, -NO₂, and CF₃; or
- R³ and R⁴: are a phenyl ring fused to the ring to which they are attached; or
- R³ and R²: are a phenyl ring fused to the ring to which they are attached.

The term "alkyl" is a straight, branched, or cyclic group of from 1 to 6 carbon atoms including but not limited to methyl, ethyl, propyl, n-propyl, isopropyl, butyl, 2-butyl, tert-butyl, pentyl, hexyl, n-hexyl, cyclopropyl, or cyclopentyl.

Preferred groups are methyl and tert-butyl.

The phenyl groups may be unsubstituted or substituted by from 1 to 5 substituents selected from hydrogen, halogen, straight, branched, or cyclic alkyl of from 1 to 6 carbons, -CF₃, -CN, -NO₂, or -OCH₃.

Halogen includes fluorine, bromine, chlorine, and iodine.

Since amino acids are amphoteric, pharmacologically compatible salts when R is hydrogen can be salts of appropriate inorganic or organic acids, for example, hydrochloric, sulphuric, phosphoric, acetic, oxalic, lactic, citric, malic, salicylic, malonic, maleic, succinic, and ascorbic. Starting from corresponding hydroxides or carbonates, salts with alkali metals or alkaline earth metals, for example, sodium, potassium, magnesium, or calcium are formed. Salts with quaternary ammonium ions can also be prepared with, for example, the tetramethyl-ammonium ion.

Prodrugs of compound I are included in the scope of the instant invention. Aminoacyl-glycolic and -lactic esters are known as prodrugs of amino acids (Wermuth C.G., *Chemistry and Industry,* 1980:433-435). The carbonyl group of the amino acids can be esterified by known means. Prodrugs and soft drugs are known in the art (Palomino E., *Drugs of the Future,* 1990;15(4):361-368).

The effectiveness of an orally administered drug is dependent upon the drug's efficient transport across the mucosal epithelium and its stability in entero-hepatic circulation. Drugs that are effective after parenteral administration but less effective orally, or whose plasma half-life is considered too short, may be chemically modified into a prodrug form.

A prodrug is a drug which has been chemically modified and may be biologically inactive at its site of action, but which may be degraded or modified by one or more enzymatic or other in vivo processes to the parent bioactive form.

This chemically modified drug, or prodrug, should have a different pharmacokinetic profile to the parent, enabling easier absorption across the mucosal epithelium, better salt formulation and/or solubility, improved systemic stability (for an increase in plasma half-life, for example). These chemical modifications may be
1) ester or amide derivatives which may be cleaved by, for example, esterases or lipases. For ester derivatives, the ester is derived from the carboxylic acid moiety of the drug molecule by known means. For amide derivatives, the amide may be derived from the carboxylic acid moiety or the amine moiety of the drug molecule by known means.
2) peptides which may be recognized by specific or nonspecific proteinases. A peptide may be coupled to the drug molecule via amide bond formation with the amine or carboxylic acid moiety of the drug molecule by known means.
3) derivatives that accumulate at a site of action through membrane selection of a prodrug form or modified prodrug form,
4) any combination of 1 to 3.

Current research in animal experiments has shown that the oral absorption of certain drugs may be increased by the preparation of "soft" quaternary salts. The quaternary salt is termed a "soft" quaternary salt since, unlike normal quaternary salts, e.g., R-N⁺(CH₃)₃, it can release the active drug on hydrolysis.

"Soft" quaternary salts have useful physical properties compared with the basic drug or its salts. Water solubility may be increased compared with other salts, such as the hydrochloride, but more important there may be an increased absorption of the drug from the intestine. Increased absorption is probably due to the fact that the "soft" quaternary salt has surfactant properties and is capable of forming micelles and unionized ion pairs with bile acids, etc., which are able to penetrate the intestinal epithelium more effectively. The prodrug, after absorption, is rapidly hydrolyzed with release of the active parent drug.

Certain of the compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms as well as the appropriate mixtures thereof. For example, the compound of Example 1 is a mixture of all four possible stereoisomers. The compound of Example 6 is one of the isomers. The configuration of the cyclohexane ring carbon centers may be R or S in these compounds where a configuration can be defined.

The radioligand binding assay using [³H]gabapentin and the α₂δ subunit derived from porcine brain tissue was used (Gee N.S., Brown J.P., Dissanayake V.U.K., Offord J., Thurlow R., Woodruff G.N., "The Novel Anti-convulsant Drug, Gabapentin, Binds to the α₂δ Subunit of a Calcium Channel," *J. Biol. Chem.,* 1996; 271: 5879-5776).

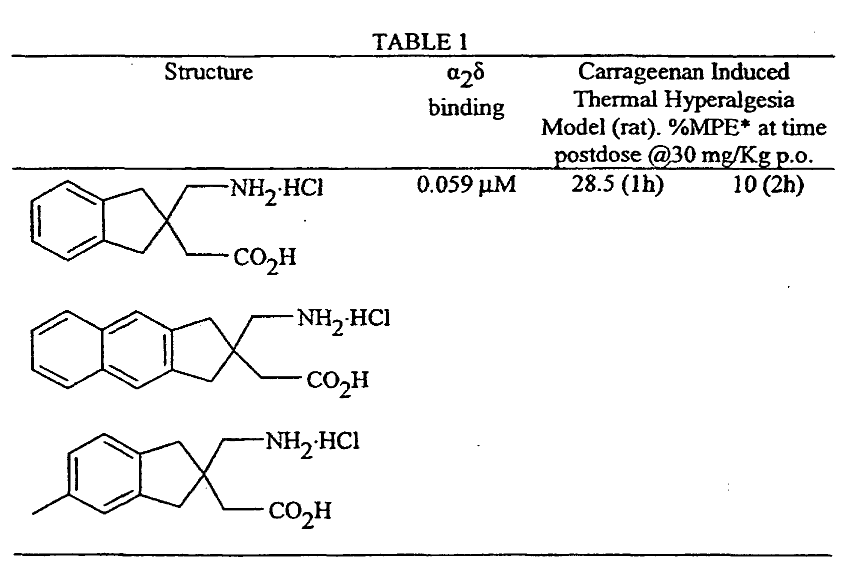

Table 1 above shows the binding affinity of the compounds of the invention to the α₂δ subunit and results from an animal model.

The compounds of the invention are compared to Neurontin®, a marketed drug effective in the treatment of such disorders as epilepsy. Neurontin® is 1-(aminomethyl)-cyclohexaneacetic acid of structural formula

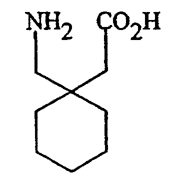

Gabapentin (Neurontin®) is about 0.10 to 0.12 µM in this assay. The compounds of the instant invention are expected, therefore, to exhibit pharmacologic properties comparable to gabapentin. For example, as agents for convulsions, anxiety, and pain.

The present invention also relates to therapeutic use of the compounds of the mimetic as agents for neurodegenerative disorders.

Such neurodegenerative disorders are, for example, Alzheimer's disease, Huntington's disease, Parkinson's disease, and Amyotrophic. Lateral Sclerosis.

The present invention also covers treating neurodegenerative disorders termed acute brain injury. These include but are not limited to: stroke, head trauma, and asphyxia.

Stroke refers to a cerebral vascular disease and may also be referred to as a cerebral vascular incident (CVA) and includes acute thromboembolic stroke. Stroke includes both focal and global ischemia. Also, included are transient cerebral ischemic attacks and other cerebral vascular problems accompanied by cerebral ischemia. A patient undergoing carotid endarterectomy specifically or other cerebrovascular or vascular surgical procedures in general, or diagnostic vascular procedures including cerebral angiography.

Other incidents are head trauma, spinal cord trauma, or injury from general anoxia, hypoxia, hypoglycemia, hypotension as well as similar injuries seen during procedures from embole, hyperfusion, and hypoxia.

The instant invention would be useful in a range of incidents, for example, during cardiac bypass surgery, in incidents of intracranial hemorrhage, in perinatal asphyxia, in cardiac arrest, and status epilepticus.

Pain refers to acute as well as chronic pain.

Acute pain is usually short-lived and is associated with hyperactivity of the sympathetic nervous system. Examples are postoperative pain and allodynia.

Chronic pain is usually defined as pain persisting from 3 to 6 months and includes somatogenic pains and psychogenic pains. Other pain is nociceptive.

Still other pain is caused by injury or infection of peripheral sensory nerves. It includes, but is not limited to pain from peripheral nerve trauma, herpes virus infection, diabetes mellitus, causalgia, plexus avulsion, neuroma, limb amputation, and vasculitis. Neuropathic pain is also caused by nerve damage from chronic alcoholism, human immunodeficiency virus infection, hypothyroidism, uremia, or vitamin deficiencies. Neuropathic pain includes, but is not limited to pain caused by nerve injury such as, for example, the pain diabetics suffer from.

Psychogenic pain is that which occurs without an organic origin such as low back pain, atypical facial pain, and chronic headache.

Other types of pain are: inflammatory pain, osteoarthritic pain, trigeminal neuralgia, cancer pain, diabetic neuropathy, restless leg syndrome, acute herpetic and postherpetic neuralgia, causalgia, brachial plexus avulsion, occipital neuralgia, gout, phantom limb, burn, and other forms of neuralgia, neuropathic and idiopathic pain syndrome.

A skilled physician will be able to determine the appropriate situation in which subjects are susceptible to or at risk of, for example, stroke as well as suffering from stroke for administration by methods of the present invention.

The compounds of the invention are also expected to be useful in the treatment of depression. Depression can be the result of organic disease, secondary to stress associated with personal loss, or idiopathic in origin. There is a strong tendency for familial occurrence of some forms of depression suggesting a mechanistic cause for at least some forms of depression. The diagnosis of depression is made primarily by quantification of alterations in patients' mood. These evaluations of mood are generally performed by a physician or quantified by a neuropsychologist using validated rating scales, such as the Hamilton Depression Rating Scale or the Brief Psychiatric Rating Scale. Numerous other scales have been developed to quantify and measure the degree of mood alterations in patients with depression, such as insomnia, difficulty with concentration, lack of energy, feelings of worthlessness, and guilt. The standards for diagnosis of depression as well as all psychiatric diagnoses are collected in the Diagnostic and Statistical Manual of Mental Disorders (Fourth Edition) referred to as the DSM-IV-R manual published by the American Psychiatric Association, 1994.

GABA is an inhibitory neurotransmitter with the central nervous system. Within the general context of inhibition, it seems likely that GABA-mimetics might decrease or inhibit cerebral function and might therefore slow function and decrease mood leading to depression.

The compounds of the instant invention may produce an anticonvulsant effect through the increase of newly created GABA at the synaptic junction. If gabapentin does indeed increase GABA levels or the effectiveness of GABA at the synaptic junction, then it could be classified as a GABA-mimetic and might decrease or inhibit cerebral function and might, therefore, slow function and decrease mood leading to depression.

The fact that a GABA agonist or GABA-mimetic might work just the opposite way by increasing mood and thus, be an antidepressant, is a new concept, different from the prevailing opinion of GABA activity heretofore.

The compounds of the instant invention are also expected to be useful in the treatment of anxiety and of panic as demonstrated by means of standard pharmacological procedures.

The compounds of the instant invention are also expected to be useful in the treatment of sleep disorders.

### MATERIAL AND METHODS

### Carrageenin-Induced Hyperalgesia

Nociceptive pressure thresholds were measured in the rat paw pressure test using an analgesimeter (Randall-Selitto method: Randall L.O. and Selitto J.J., "A method for measurement of analgesic activity on inflamed tissue," *Arch. Int. Pharmacodyn.,* 1957;4:409-419). Male Sprague-Dawley rats (70-90 g) were trained on this apparatus before the test day. Pressure was gradually applied to the hind paw of each rat and nociceptive thresholds were determined as the pressure (g) required to elicit paw withdrawal. A cutoff point of 250 g was used to prevent any tissue damage to the paw. On the test day, two to three baseline measurements were taken before animals were administered 100 µL of 2% carrageenin by intraplantar injection into the right hind paw. Nociceptive thresholds were taken again 3 hours after carrageenin to establish that animals were exhibiting hyperalgesia. Animals were dosed with either gabapentin (3-300 mg, s.c.), morphine (3 mg/kg, s.c.) or saline at 3.5 hours after carrageenin and nociceptive thresholds were examined at 4, 4.5, and 5 hours postcarrageenin.

(R)-2-Aza-spiro[4.5]decane-4-carboxylic acid hydrochloride was tested in the above carrageenan-induced hyperalgesia model. The compound was dosed orally at 30 mg/kg, and 1 hour postdose gave a percent of maximum possible effect (MPE) of 53%. At 2 hours postdose, it gave only 4.6% of MPE.

### Semicarbazide-Induced Tonic Seizures

Tonic seizures in mice are induced by subcutaneous administration of semicarbazide (750 mg/kg). The latency to the tonic extension of forepaws is noted. Any mice not convulsing within 2 hours after semicarbazide are considered protected and given a maximum latency score of 120 minutes.

### Animals

Male Hooded Lister rats (200-250 g) are obtained from Interfauna (Huntingdon, UK) and male TO mice (20-25 g) are obtained from Bantin and Kingman (Hull, UK). Both rodent species are housed in groups of six. Ten Common Marmosets (Callithrix Jacchus) weighing between 280 and 360 g, bred at Manchester University Medical School (Manchester, UK) are housed in pairs. All animals are housed under a 12-hour light/dark cycle (lights on at 07.00 hour) and with food and water ad libitum.

### Drug Administration

Drugs are administered either intraperitoneally (IP) or subcutaneously (SC) 40 minutes before the test in a volume of I mL/kg for rats and marmosets and 10 mL/kg for mice.

### Mouse Light/Dark Box

The apparatus is an open-topped box, 45 cm long, 27 cm wide, and 27 cm high, divided into a small (2/5) and a large (3/5) area by a partition that extended 20 cm above the walls (Costall B., et al., "Exploration of mice in a black and white box: validation as a model of anxiety," *Pharmacol. Biochem. Behav.,* 1989;32:777-785 ).

There is a 7.5 × 7.5 cm opening in the center of the partition at floor level. The small compartment is painted black and the large compartment white. The white compartment is illuminated by a 60-W tungsten bulb. The laboratory is illuminated by red light. Each mouse is tested by placing it in the center of the white area and allowing it to explore the novel environment for 5 minutes. The time spent in the illuminated side is measured (Kilfoil T., et al., "Effects of anxiolytic and anxiogenic drugs on exploratory activity in a simple model of anxiety in mice," *Neuropharmacol.,* 1989,28:901-905).

### Rat Elevated X-Maze

A standard elevated X-maze (Handley S.L., et al., "Effects of alpha-adrenoceptor agonists and antagonists in a maze-exploration model of 'fear'-motivated behavior," *Naunyn-Schiedeberg's Arch. Pharmacol.,* 1984;327:1-5), was automated as previously described (Field, et al., "Automation of the rat elevated X-maze test of anxiety.*" Br. J Pharmacol.,* 1991; 102 (Suppl.): 304P). The animals are placed on the center of the X-maze facing one of the open arms. For determining anxiolytic effects the entries and time spent on the end half sections of the open arms is measured during the 5-minute test period (Costall, et al., "Use of the elevated plus maze to assess anxiolytic potential in the rat," *Br. J Pharmacol.,* 1989; 96 (Suppl.): 312p).

### Marmoset Human Threat Test

The total number of body postures exhibited by the animal towards the threat stimulus (a human standing approximately 0.5 m away from the marmoset cage and staring into the eyes of the marmoset) is recorded during the 2-minute test period. The body postures scored are slit stares, tail postures, scent marking of the cage/perches, piloerection, retreats, and arching of the back. Each animal is exposed to the threat stimulus twice on the test day before and after drug treatment. The difference between the two scores is analyzed using one-way analysis of variance followed by Dunnett's 1-test. All drug treatments are carried out SC at least 2 hours after the first (control) threat. The pretreatment time for each compound is 40 minutes.

### Rat Conflict Test

Rats are trained to press levers for food reward in operant chambers. The schedule consists of alternations of four 4-minute unpunished periods on variable interval of 30 seconds signaled by chamber lights on and three 3-minute punished periods on fixed ratio 5 (by footshock concomitant to food delivery) signaled by chamber lights off. The degree of footshock is adjusted for each rat to obtain approximately 80% to 90% suppression of responding in comparison with unpunished responding. Rats receive saline vehicle on training days.

### DBA2 Mouse Model of Anticonvulsant Efficacy

All procedures were carried out in compliance with the NIH Guide for the Care and Use of Laboratory Animals under a protocol approved by the Parke-Davis Animal Use Committee. Male DBA/2 mice, 3 to 4 weeks old were obtained from Jackson Laboratories Bar Harbour, Maine. Immediately before anticonvulsant testing, mice were placed upon a wire mesh, 4 inches square, suspended from a steel rod. The square was slowly inverted through 180° and mice observed for 30 seconds. Any mouse falling from the wire mesh was scored as ataxic (Coughenour L.L., McLean J.R., Parker R.B., "A new device for the rapid measurement of impaired motor function in mice," *Pharm. Biochem. Behav.,* 1977; 6(3): 351-3). Mice were placed into an enclosed acrylic plastic chamber (21 cm height, approximately 30 cm diameter) with a high-frequency speaker (4 cm diameter) in the center of the top lid. An audio signal generator (Protek model B-810) was used to produce a continuous sinusoidal tone that was swept linearly in frequency between 8 kHz and 16 kHz once each 10 msec. The average sound pressure level (SPL) during stimulation was approximately 100 dB at the floor of the chamber. Mice were placed within the chamber and allowed to acclimatize for one minute. DBA/2 mice in the vehicle-treated group responded to the sound stimulus (applied until tonic extension occurred, or for a maximum of 60 sec) with a characteristic seizure sequence consisting of wild running followed by clonic seizures, and later by tonic extension, and finally by respiratory arrest and death in 80% or more of the mice. In vehicle-treated mice, the entire sequence of seizures to respiratory arrest lasts approximately 15 to 20 seconds. The incidence of all the seizure phases in the drug-treated and vehicle-treated mice was recorded, and the occurrence of tonic seizures were used for calculating anticonvulsant ED₅₀ values by probit analysis (Litchfield J.T., Wilcoxon F. "A simplified method for evaluating dose-effect experiments," *J. Pharmacol.,* 1949;96:99-113). Mice were used only once for testing at each dose point. Groups of DBA/2 mice (n = 5-10 per dose) were tested for sound-induced seizure responses 2 hours (previously determined time of peak effect) after given drug orally. All drugs in the present study were dissolved in distilled water and given by oral gavage in a volume of 10 mL/kg of body weight. Compounds that are insoluble will be suspended in 1% carboxymethocellulose. Doses are expressed as weight of the active drug moiety.

The compounds of the instant invention are also expected to be useful in the treatment of pain and phobic disorders (*Am. J. Pain Manag.,* 1995;5:7-9).

The compounds of the instant invention are also expected to be useful in treating the symptoms of manic, acute or chronic, single upside, or recurring depression. They are also expected to be useful in treating and/or preventing bipolar disorder (United States Patent Number 5,510,381).

The compounds are useful in sleep disorders. The method for evaluating them is fully described in Drug Development Res., 1988;14:151-159.

Sleep disorders are disturbances that affect the ability to fall and/or stay asleep, that involve sleeping too much, or that result in abnormal behavior associated with sleep. The sleep disorders include, for example, insomnia, drug-associated sleeplessness, hypersomnia, narcolepsy, insomnia syndromes, and parasomnias.

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Also, the compounds of the present invention can be administered by inhalation, for example, intranasally. Additionally, the compounds of the present invention can be administered transdermally. It will be obvious to those skilled in the art that the following dosage forms may comprise as the active component, either a compound of Formula I or a corresponding pharmaceutically acceptable salt of a compound of Formula I.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax and cocoa butter. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose. and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners and solubilizing agents.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form.

The quantity of active component in a unit dose preparation may be varied or adjusted from 0.1 mg to 1 g according to the particular application and the potency of the active component. In medical use the drug may be administered three times daily as, for example, capsules of 100 or 300 mg. The composition can, if desired, also contain other compatible therapeutic agents.

In therapeutic use, the compounds utilized in the pharmaceutical method of this invention are administered at the initial dosage of 0.01 mg to 100 mg/kg daily. A daily dose range of 0.01 mg to 100 mg/kg is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day, if desired.

The following examples are illustrative of the instant invention; they are not intended to limit the scope.

### EXAMPLE 1

### Synthesis of the 2-indanone Compound (5)

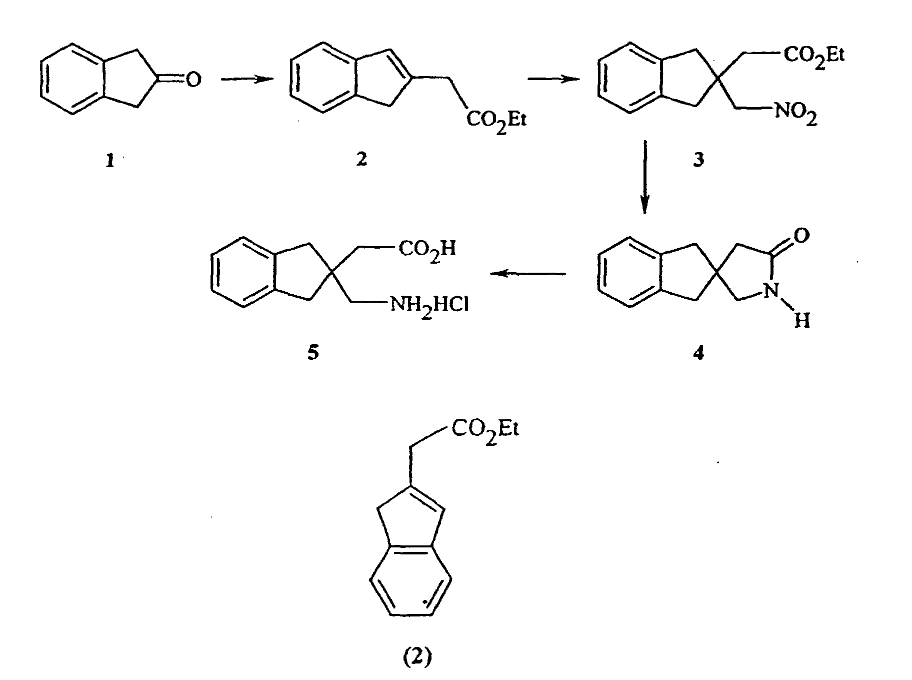

### Synthesis of compound (2)

Triethylphosphonoacetate (5.5 mL, 27.8 mmol) was slowly added (over 15 minutes) to a suspension of sodium hydride (1.06 g, 26.6 mmol) in THF (60 mL) at 0°C under nitrogen. Gas was evolved and the solution went clear. Next, 2-indanone **(1)** (335 g, 25.3 mmol in THF (10 mL + 5 mL) was added, and the solution was allowed to warm to room temperature with stirring over 3 hours, after which the reaction was diluted with water (150 mL) and extracted with ether (2 × 200 mL), then dried (MgSO₄). The organic phase was concentrated in vacuo. Column chromatography (40% ethyl acetate in heptane) gave 4.45 g (87%) of the product as an oil.
¹H NMR (CDCl₃) δ = 7.40 (4H, m), 7.31 (1H, d, *J* 7.6), 7.25 (1H, t, *J* 7.6), 7.15 (1H, t, *J* 7.4), 6.7 (1H, br s), 4.18 (2H, q, *J* 7.1), 3.52 (2H, s), 3.45 (2H, s), 3.45 (2H, s), 1.28 (3H, t, *J* 7.2).
I.R (thin film) (cm⁻¹) ν = 2981, 1782, 1734, 1613, 1461, 1369, 1174, 1029, 753.

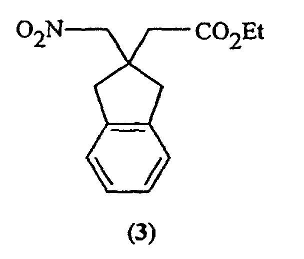

### Synthesis of compound (3)

Nitromethane (2.1 mL, 38.88 mL) was added to a stirring solution of (2) (2.00 g, 9.9 mmol) and TBAF (1.0M in THF, 11.6 mL, 11.6 mmol ) in THF (14 mL). The reaction was heated (oil bath at 60°C) for 5 hours, after which the reaction was diluted with ethyl acetate (100 mL) and washed with 1M HCl (30 mL) and saturated brine (40 mL). The organic phase was then dried (MgSO₄) and concentrated in vacuo. Column chromatography (10% ethyl acetate in heptane) gave an 8:1 mixture of starting material/product as a yellow oil. The product was further purified by column chromatography (10% ethyl acetate in heptane) to yield 0.21 g, (8%) of the product as a yellow oil. Recovered Starting material = 1.3904 g, 69%.
¹H NMR (CDCl₃) δ = 7.26 (4H, m), 4.75 (2H, s), 4.15 (2H, q, *J* 7.2), 3.10 (2H, d, *J* 16.4), 2.85 (2H, d, *J* 16), 2.71 (2H, s), 1.26 (3H, t, *J* 7.2).

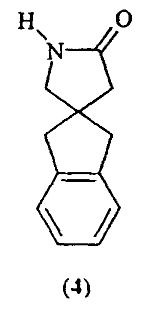

### Synthesis of compound (4)

Compound (3) (0.45 g, 1.7 mmol) and a small spatula of nickel sponge catalyst, in methanol (100 mL) underwent hydrogenation at 30°C and a pressure of 78 psi, for 5 hours (before use the catalyst was washed several times, first with water and then with methanol.), after which the reaction mixture was filtered through celite and the resulting solution concentrated under reduced pressure to give a yellow/orange solid. The reaction gave a quantitative yield.
¹H NMR (CDCl₃) δ = 7.23-7.10 (4H, m), 5.48 (1H, br s), 3.35 (2H, s), 3.03 (4H, s), 2.43 (2H, s), m/z (ES⁺) 234, corresponding to the M+1 at 100%.

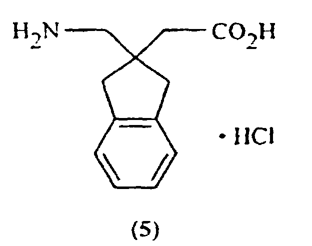

### Synthesis of compound (5)

6M HCl (15 mL, 0.09 mol) was added to a solution of (4) (0.33 g, 1.4 mmol) in dioxane (5 mL) and the solution heated to reflux (oil bath at 110°C) for 4 hours. After the reaction had cooled, it was diluted with water (20 mL), the mixture was washed with dichloromethane (3 × 20 mL), and then concentrated in vacuo. The resulting yellow oil was washed with ethyl acetate and acetonitrile, then further dried on the rotary evaporator to yield 0.30 g (87%) of product as a white powder.
¹H NMR (D₂O) δ = 7.35 (4H, m), 3.35 (2H, s), 3.11 (4H, s), 2.77 (2H, s). m/z (ES⁺) 206, corresponding to M+1 at 31% and 411 corresponding to 2M + 1 at 4%. CHN;- C₁₂H₁₅O₂N·HCl·0.5 H₂O
Expected;- C, 57.19%; H; 6.63%; N, 5.59%.
Obtained;- C, 57.49%; H, 6.83%; N, 5.59%.

## Claims

1. A compound of Formula I 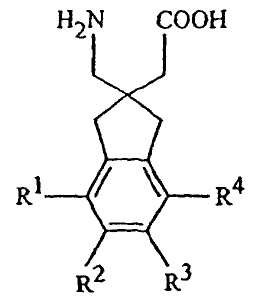 or a pharmaceutically acceptable salt thereof wherein:
R¹, R², R³, and R⁴ are each independently absent or selected from
hydrogen,
halogen,
-CN,
-NO₂,
-CF₃,
-OH,
-SH,
-Z-A wherein Z is -O-, -S-, or (CH₂)ₙ wherein n is an integer of from 0 to 6 and A is a straight, branched, or cyclic alkyl of from 1 to 6 carbons or phenyl substituted with from 1 to 5 substituents selected from H, F, Cl, Br, I, -CN, -NO₂, -CF₃, -OCH₃, or a straight, branched, or cyclic alkyl of from 1 to 6 carbons, 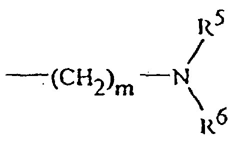 wherein m is an integer of from 0 to 6, R⁵
is H, a straight or branched or cyclic alkyl of from 1 to 6 carbons or phenyl substituted with from 1 to 5 groups each independently selected from H, F, Cl, Br, I, -CN, -NO₂, and -CF₃, and R⁶ is H, straight or branched or cyclic alkyl of from 1 to 6 carbons; 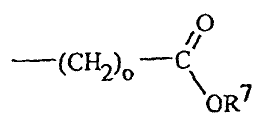 wherein o is an integer of from 0 to 6,
and R⁷ is H, straight chain, branched, or cyclic alkyl of from 1 to 6 carbons, or phenyl substituted with from 1 to 5 groups each independently selected from H, F, Cl, Br, I, -CN, -NO₂, and CF₃; or
R³ and R⁴ are a phenyl ring fused to the ring to which they are attached; or
R³ and R² are a phenyl ring fused to the ring to which they are attached.

2. A compound according to Claim 1 wherein R¹ to R⁴ are absent.

3. A compound according to Claim 1 which is selected from:
(2-aminomethyl-indan-2-yl)-acetic acid;
(2-aminomethyl-4,7-dimethyl-indan-2-yl)-acetic acid;
(2-aminomethyl-5,6-dimethyl-indan-2-yl)-acetic acid;
(2-aminomethyl-4-methyl-indan-2-yl)-acetic acid;
(2-aminomethyl-5-methyl-indan-2-yl)-acetic acid;
(4,7-dichloro-2-aminomethyl-indan-2-yl)-acetic acid;
(5,6-dichloro-2-aminomethyl-indan-2-yl)-acetic acid;
(4-chloro-2-aminomethyl-indan-2-yl)-acetic acid;
(5-chloro-2-aminomethyl-indan-2-yl)-acetic acid;
(2-aminomethyl-2,3-dihydro-1H-cyclopenta[b]naphthalen-2-yl)-acetic acid;
(2-Aminomethyl-2,3-dihydro-1H-cyclopenta[a]naphthalen-2-yl)-acetic acid;
(2-Aminomethyl-4,7-dimethoxy-indan-2-yl)-acetic acid;
(2-Aminomethyl-5,6-dimethoxy-indan-2-yl)-acetic acid;
(2-Aminomethyl-4-methoxy-indan-2-yl)-acetic acid;
(2-Aminomethyl-5-methoxy-indan-2-yl)-acetic acid;
(2-Aminomethyl-4,7-dibromo-indan-2-yl)-acetic acid;
(2-Aminomethyl-5,6-dibromo-indan-2-yl)-acetic acid;
(2-Aminomethyl-4-bromo-indan-2-yl)-acetic acid;
(2-Aminomethyl-5-bromo-indan-2-yl)-acetic acid;
(4-Amino-2-aminomethyl-indan-2-yl)-acetic acid; -
(5-Amino-2-aminomethyl-indan-2-yl)-acetic acid;
2-Aminomethyl-2-carboxymethyl-indan-4-carboxylic acid;
2-Aminomethyl-2-carboxymethyl-indan-5-carboxylic acid;
2-Aminomethyl-2-carboxymethyl-indan-4-carboxylic acid methyl ester,
2-Aminomethyl-2-carboxymethyl-indan-5-carboxylic acid methyl ester;
(2-Aminomethyl-4-methylamino-indan-2-yl)-acetic acid;
(2-Aminomethyl-5-methylamino-indan-2-yl)-acetic acid;
(2-Aminomethyl-4-cyano-indan-2-yl)-acetic acid;
(2-Aminomethyl-5-cyano-indan-2-yl)-acetic acid;
(2-Aminomethyl-4-trifluoromethyl-indan-2-yl)-acetic acid;
(2-Aminomethyl-1,7-bis-trifluoromethyl-indan-2-yl)-acetic acid;
(2-Aminomethyl-5-trifluoromethyl-indan-2-yl)-acetic acid;
(2-Aminomethyl-5,6-bis-trifluoromethyl-indan-2-yl)-acetic acid;
(2-Aminomethyl-4-hydroxy-indan-2-yl)-acetic acid;
(2-Aminomethyl-5-hydroxy-indan-2-yl)-acetic acid;
(2-Aminomethyl-5-phenyl-indan-2-yl)-acetic acid;
(2-Aminomethyl-4-phenyl-indan-2-yl)-acetic acid;
(2-Aminomethyl-5,6-diphenyl-indan-2-yl)-acetic acid;
(2-Aminomethyl-4,7-diphenyl-indan-2-yl)-acetic acid;
(2-Aminomethyl-4,5-diphenyl-indan-2-yl)-acetic acid;
(2-Aminomethyl-4-isopropyl-indan-2-yl)-acetic acid;
(2-Aminomethyl-5-isopropyl-indan-2-yl)-acetic acid;
(2-Aminomethyl-4,7-diisopropyl-indan-2-yl)-acetic acid;
(2-Aminomethyl-4-isobutyl-indan-2-yl)-acetic acid;
(2-Aminomethyl-5-isobutyl-indan-2-yl)-acetic acid;
2-Aminomethyl-4-dimethylamino-indan-2-yl)-acetic acid;
2-Aminomethyl-5-dimethylamino-indan-2-yl)acetic acid;
(2-Aminomethyl-4-iodo-indan-2-yl)-acetic acid;
(2-Aminomethyl-5-iodo-indan-2-yl)-acetic acid;
(2-Aminomethyl-4-nitro-indan 2-yl)-acetic acid; and
(2-Aminomethyl-5-nitro-indan-2-yl)-acetic acid

4. A pharmaceutical composition comprising a therapeutically effective amount of a compound according to any one of Claims 1 to 3 and a pharmaceutically acceptable carrier.

5. Use of a compound according to any one of Claims 1 to 3 in the manufacture of a medicament for treating epilepsy.

6. Use of a compound according to any one of Claims 1 to 3 in the manufacture of a medicament for treating faintness attacks, hypokinesia, and cranial disorders.

7. Use of a compound according to any one of Claims 1 to 3 in the manufacture of a medicament for treating neurodegenerative disorders.

8. Use of a compound according to any one of Claims 1 to 3 in the manufacture of a medicament for treating depression.

9. Use of a compound according to any one of Claims 1 to 3 in the manufacture of a medicament for treating anxiety.

10. Use of a compound according to any one of Claims 1 to 3 in the manufacture of a medicament for treating panic.

11. Use of a compound according to any one of Claims 1 to 3 in the manufacture of a medicament for treating pain.

12. Use of a compound according to any one of Claims 1 to 3 in the manufacture of a medicament for treating neuropathological disorders.

13. Use of a compound according to any one of Claims 1 to 3 in the manufacture of a medicament for treating sleep disorders.

14. Compound of any one of Claims 1 to 3 for use in medicine.

## Patentansprüche

1. Verbindung mit der Formel I 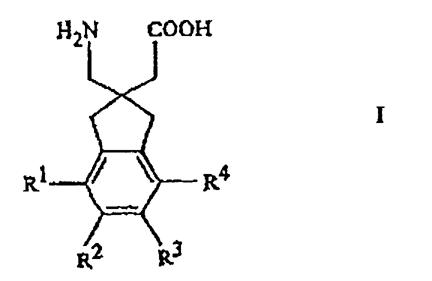 oder pharmazeutisch annehmbares Salz von ihr, wobei
R¹, R², R³ und R⁴ jeweils unabhängig voneinander nicht vorhanden sind oder ausgewählt sind aus
Wasserstoff,
Halogen,
-CN,
-NO₂,
-CF₃,
-OH,
-SH,
- Z-A, wobei Z ist -O-, -S- oder (CH₂)ₙ, wobei n eine ganze Zahl von 0 bis 6 ist, und A ist ein linearer, verzweigter oder cyclischer Alkylrest mit 1 bis 6 Kohlenstoffatomen oder ein Phenylrest, der mit 1 bis 5 Substituenten substituiert ist, die ausgewählt sind aus H, F, Cl, Br, I, -CN, -NO₂, -CF₃, - OCH₃ oder einem linearen, verzweigten oder cyclischen Alkylrest mit 1 bis 6 Kohlenstoffatomen; 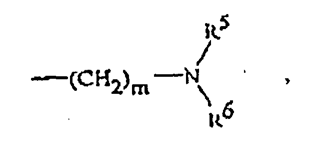 wobei m eine ganze Zahl von 0 bis 6 ist, R⁵ ist H, ein linearer oder verzweigter oder cyclischer Alkylrest mit 1 bis 6 Kohlenstoffatomen oder ein Phenylrest, der mit 1 bis 5 Gruppen substituiert ist, die jeweils unabhängig voneinander ausgewählt sind aus H, F, Cl, Br, I, -CN, -NO₂ und -CF₃, und R⁶ ist H, ein linearer oder verzweigter oder cyclischer Alkylrest mit 1 bis 6 Kohlenstoffatomen; 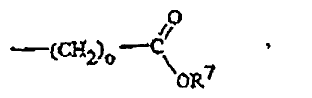 wobei o eine ganze Zahl von 0 bis 6 ist, und R⁷ ist H, ein linearer, verzweigter oder cyclischer Alkylrest mit 1 bis 6 Kohlenstoffatomen oder ein Phenylrest, der mit 1 bis 5 Gruppen substituiert ist, die jeweils unabhängig voneinander ausgewählt sind aus H, F, Cl, Br, I, -CN, -NO₂ und -CF₃; oder
R³ und R⁴ sind ein Phenylring, der mit dem Ring fusioniert ist, an dem sie angebracht sind;
oder
R³ und R² sind ein Phenylring, der mit dem Ring fusioniert ist, an dem sie angebracht sind.

2. Verbindung gemäß Anspruch 1, wobei R¹ bis R⁴ nicht vorhanden sind.

3. Verbindung gemäß Anspruch 1, die ausgewählt ist aus
(2-Aminomethyl-indan-2-yl)-essigsäure,
(2-Aminomethyl-4,7-dimethyl-indan-2-yl)-essigsäure,
(2 Aminomethyl-5,6-dimethyl-indan-2-yl)-essigsäure,
(2-Aminomethyl-4-methyl-indan-2-yl)-essigsäure,
(2-Aminomethyl-5-methyl-indan-2-yl)-essigsäure,
(4,7-Dichlor-2-aminomethyl-indan-2-yl)-essigsäure,
(5,6-Dichlor-2-aminomethyl-indan-2-yl)-essigsäure,
(4-Chlor-2-aminomethyl-indan-2-yl)-essigsäure,
(5-Chlor-2-aminomethyl-indan-2-yl)-essigsäure,
(2-Aminomethyl-2,3-dihydro-1H-cyclopenta[b]naphthalin-2-yl)-essigsäure,
(2-Aminomethyl-2,3-dihydro-1H-cyclopenta[a]naphthalin-2-yl)-essigsäure,
(2-Aminomethyl-4,7-dimethoxy-indan-2-yl)-essigsäure,
(2-Aminomethyl-5,6-dimethoxy-indan-2-yl)-essigsäure,
(2-Aminomethyl-4-methoxy-indan-2-yl)-essigsäure,
(2-Aminomethyl-5-methoxy-indan-2-yl)-essigsäure,
(2 Aminomethyl-4,7-dibrom-indan-2-yl)-essigsäure,
(2-Aminomethyl-5,6-dibrom-indan-2-yl)-essigsäure,
(2-Aminomethy)-4-brom-indan-2-yl)-essigsäure,
(2-Aminomethyl-5-brom-indan-2-yl)-essigsäure,
(4-Amino-2-aminomethyl-indan-2-yl)-essigsäure,
(5-Amino-2-aminomethy)-indan-2-yl)-essigsäure,
2-Aminomethyl-2-carboxymethyl-indan-4-carbonsäure,
2-Aminomethyl-2-carboxymethyl-indan-5-carbonsäure,
2-Aminomethyl-2-carboxymethyl-indan-4-carbonsäuremethylester,
2-Aminomethyl-2-carboxymethyl-indan-5-carbonsäuremethylester,
(2-Aminomethyl-4-methylamino-indan-2-yl)-essigsäure,
(2-Aminomethyl-5-methylamino-indan-2-yl)-essigsäure,
(2-Aminomethyl-4-cyano-indan-2-yl)-essigsäure,
(2-Aminomethyl-5-cyano-indan-2-yl)-essigsäure,
(2-Aminomethyl-4-trifluormethyl-indan-2-yl)-essigsäure,
(2-Aminomethyl-1,7-bis-trifluomethyl-indan-2-yl)-essigsäure,
(2-Aminomethyl-5-trifluormethyl-indan-2-yl)-essigsäure,
(2-Aminomethyl-5,6-bis-trifluormethyl-indan-2-yl)-essigsäure,
(2-Aminomethyl-4-hydroxy-indan-2-yl)-essigsäure,
(2-Aminomethyl-5-hydroxy-indan-2-yl)-essigsäure,
(2-Aminomethyl-5-phenyl-indan-2-yl)-essigsäure,
(2-Aminomethyl-4-phenyl-indan-2-yl)-essigsäure,
(2-Aminomethyl-5,6-diphenyl-indan-2-yl)-essigsäure,
(2-Aminomethyl-4,7-diphenyl-indan-2-yl)-essigsäure,
(2-Aminomethyl-4,5-diphenyl-indan-2-yl)-essigsäure,
(2-Aminomethyl-4-isopropyl-indan-2-yl)-essigsäure,
(2-Aminomethyl-5-isopropyl-indan-2-yl)-essigsäure,
(2-Aminomethyl-4,7-diisopropyl-indan-2-yl)-essigsäure,
(2-Aminomethyl-4-isobutyl-indan-2-yl)-essigsäure,
(2-Aminomethyl-5-isobutyl-indan-2-yl)-essigsäure,
(2-Aminomethyl-4-dimethylamino-indan-2-yl)-essigsäure,
(2-Aminomethy)-5-dimethylamino-indan-2-yl)-essagsäure,
(2-Aminomethyl-4-iod-indan-2-yl)-essigsäure,
(2-Aminomethyl-5-iod-indan-2-yl)-essigsäure,
(2-Aminomethyl-4-nitro-indan-2-yl)-essigsäure, und
(2-Aminomethyl-6-nitro-indan-2-yl)-essigsäure.

4. Pharmazeutische Zusammensetzung, die eine therapeutisch wirksame Menge einer Verbindung gemäß einem beliebigen der Ansprüche 1 bis 3 und einen pharmazeutisch annehmbaren Träger umfasst.

5. Verwendung einer Verbindung gemäß einem beliebigen der Ansprüche 1 bis 3 bei der Herstellung eines Medikaments zur Behandlung der Epilepsie.

6. Verwendung einer Verbindung gemäß einem beliebigen der Ansprüche 1 bis 3 bei der Herstellung eines Medikaments zur Behandlung von Schwächeanfällen, einer Hypokinese sowie von Schädelerkrankungen.

7. Verwendung einer Verbindung gemäß einem beliebigen der Ansprüche 1 bis 3 bei der Herstellung eines Medikaments zur Behandlung neurodegenerativer Erkrankungen.

8. Verwendung einer Verbindung gemäß einem beliebigen der Ansprüche 1 bis 3 bei der Herstellung eines Medikaments zur Behandlung von Depressionen.

9. Verwendung einer Verbindung gemäß einem beliebigen der Ansprüche 1 bis 3 bei der Herstellung eines Medikaments zur Behandlung von Angstpsychosen.

10. Verwendung einer Verbindung gemäß einem beliebigen der Ansprüche 1 bis 3 bei der Herstellung eines Medikaments zur Behandlung von Panikanfallen.

11. . Verwendung einer Verbindung gemäß einem beliebigen der Ansprüche 1 bis 3 bei der Herstellung eines Medikaments zur Behandlung von Schmerzen.

12. Verwendung einer Verbindung gemäß einem beliebigen der Ansprüche 1 bis 3 bei der Herstellung eines Medikaments zur Behandlung neuropathologischer Erkrankungen.

13. Verwendung einer Verbindung gemäß einem beliebigen der Ansprüche 1 bis 3 bei der Herstellung eines Medikaments zur Behandlung von Schlafstörungen.

14. Verbindung nach einem beliebigen der Ansprüche 1 bis 3 zur Verwendung in der Medizin.

## Revendications

1. Composé de formule I 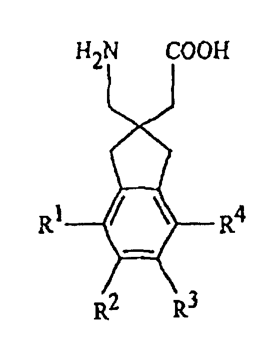 ou un sel pharmaceutiquement acceptable de celui-ci, où :
R¹, R², R³ et R⁴ sont chacun indépendamment absents ou choisis parmi :
l'hydrogène,
un halogène,
- CN,
-NO₂,
-CF₃,
- OH,
- SH,
- Z-A, où Z est -O-, -S-, ou (CH₂)ₙ, où n est un entier allant de 0 à 6 et A est un alkyle à chaîne droite, ramifiée ou cyclique ayant de 1 à 6 atomes de carbone ou un phényle substitué par de 1 à 5 substituants choisis parmi H, F, Cl, Br, I, -CN, -NO₂, -CF₃, -OCH₃, ou un alkyle à chaîne droite, ramifiée ou cyclique ayant de 1 à 6 atomes de carbone ; 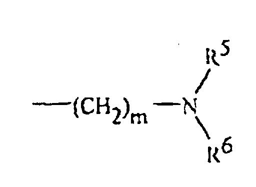 où m est un entier allant de 0 à 6, R⁵ est H, un alkyle à chaîne droite ou ramifiée ou cyclique ayant de 1 à 6 atomes de carbone ou un phényle substitué par de 1 à 5 groupes choisis chacun indépendamment parmi H, F, Cl, Br, I, -CN, -NO₂, et -CF₃, et R⁶ est H, un alkyle à chaîne droite ou ramifiée ou cyclique ayant de 1 à 6 atomes de carbone ; 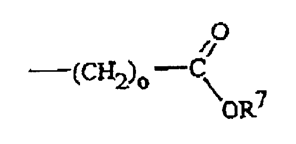 où o est un entier allant de 0 à 6, et R⁷ est H, un alkyle à chaîne droite, ramifiée ou cyclique ayant de 1 à 6 atomes de carbone, ou un phényle substitué par de 1 à 5 groupes choisis chacun indépendamment parmi H, F, Cl, Br, I, -CN, -NO₂ et CF₃ ;
ou
R³ et R⁴ sont un cycle phényle accolé au cycle auquel ils sont attachés ; ou
R³ et R² sont un cycle phényle accolé au cycle auquel ils sont attachés.

2. Composé selon la revendication 1, dans lequel R¹ et R⁴ sont absents.

3. Composé selon la revendication 1, qui est choisi parmi :
l'acide (2-aminométhyl-indan-2-yl)-acétique ;
l'acide (2-aminométhyl-4,7-diméthyl-indan-2-yl)-acétique ;
l'acide (2-aminométhyl-5,6-diméthyl-indan-2-yl)-acétique ;
l'acide (2-aminométhyl-4-méthyl-indan-2-yl)-acétique ;
l'acide (2-aminométhyl-5-méthyl-indan-2-yl)-acétique ;
l'acide (4,7-dichloro-2-aminométhyl-indan-2-yl)-acétique ;
l'acide (5,6-dichloro-2-aminométhyl-indan-2-yl)-acétique ;
l'acide (4-chloro-2-aminométhyl-indan-2-yl)-acétique ;
l'acide (5-chloro-2-aminométhyl-indan-2-yl)-acétique ;
l'acide (2-aminométhyl-2,3-dihydro-1H-cyclopenta[b]naphtalèn-2-yl)-acétique ;
l'acide (2-aminométhyl-2,3-dihydro-1H-cyclopenta[a]naphtalèn-2-yl)-acétique ;
l'acide (2-aminométhyl-4,7-diméthoxy-indan-2-yl)-acétique ;
l'acide (2-aminométhyl-5,6-diméthoxy-indan-2-yl)-acétique ;
l'acide (2-aminométhyl-4-méthoxy-indan-2-yl)-acétique ;
l'acide (2-aminométhyl-5-méthoxy-indan-2-yl)-acétique ;
l'acide (2-aminométhyl-4,7-dibromo-indan-2-yl)-acétique ;
l'acide (2-aminométhyl-5,6-dibromo-indan-2-yl)-acétique ;
l'acide (2-aminométhyl-4-bromo-indan-2-yl)-acétique ;
l'acide (2-aminométhyl-5-bromo-indan-2-yl)-acétique ;
l'acide (4-amino-2-aminométhyl-indan-2-yl)-acétique ;
l'acide (5-amino-2-aminométhyl-indan-2-yl)-acétique ;
l'acide 2-aminométhyl-2-carboxyméthyl-indan-4-carboxylique ;
l'acide 2-aminométhyl-2-carboxyméthyl-indan-5-carboxylique ;
l'ester méthylique de l'acide 2-aminométhyl-2-carboxyméthyl-indan-4-carboxylique ;
l'ester méthylique de l'acide 2-aminométhyl-2-carboxyméthyl-indan-5-carboxylique ;
l'acide (2-aminométhyl-4-méthylamino-indan-2-yl)-acétique ;
l'acide (2-aminométhyl-5-méthylamino-indan-2-yl)-acétique ;
l'acide (2-aminométhyl-4-cyano-indan-2-yl)-acétique ;
l'acide (2-aminométhyl-5-cyano-indan-2-yl)-acétique ;
l'acide (2-aminométhyl-4-trifluorométhylindan-2-yl)-acétique ;
l'acide (2-aminométhyl-4,7-bis-trifluorométhyl-indan-2-yl)-acétique ;
l'acide (2-aminométhyl-5-trifluorométhyl-indan-2-yl)-acétique ;
l'acide (2-aminométhyl-5,6-bis-trifluorométhyl-indan-2-yl)-acétique ;
l'acide (2-aminométhyl-4-hydroxy-indan-2-yl)-acétique ;
l'acide (2-aminométhyl-5-hydroxy-indan-2-yl)-acétique ;
l'acide (2-aminométhyl-5-phényl-indan-2-yl)-acétique ;
l'acide (2-aminométhyl-4-phényl-indan-2-yl)-acétique ;
l'acide (2-aminométhyl-5,6-diphényl-indan-2-yl)-acétique ;
l'acide (2-aminométhyl-4,7-diphényl-indan-2-yl)-acétique ;
l'acide (2-aminométhyl-4,5-diphényl-indan-2-yl)-acétique ;
l'acide (2-aminométhyl-4-isopropyl-indan-2-yl)-acétique ;
l'acide (2-aminométhyl-5-isopropyl-indan-2-yl)-acétique ;
l'acide (2-aminométhyl-4,7-diisopropyl-indan-2-yl)-acétique ;
l'acide (2-aminométhyl-4-isobutyl-indan-2-yl)-acétique ;
l'acide (2-aminométhyl-5-isobutyl-indan-2-yl)-acétique ;
l'acide (2-aminométhyl-4-diméthylamino-indan-2-yl)-acétique ;
l'acide (2-aminométhyl-5-diméthylamino-indan-2-yl)-acétique ;
l'acide (2-aminométhyl-4-iodo-indan-2-yl)-acétique ;
l'acide (2-aminométhyl-5-iodo-indan-2-yl)-acétique ;
l'acide (2-aminométhyl-4-nitro-indan-2-yl)-acétique ; et
l'acide (2-aminométhyl-5-nitro-indan-2-yl)-acétique.

4. Composition pharmaceutique comprenant une quantité thérapeutiquement efficace d'un composé selon l'une quelconque des revendications 1 à 3, et un véhicule pharmaceutiquement acceptable.

5. Utilisation d'un composé selon l'une quelconque des revendications 1 à 3, dans la fabrication d'un médicament destiné à traiter l'épilepsie.

6. Utilisation d'un composé selon l'une quelconque des revendications 1 à 3, dans la fabrication d'un médicament destiné à traiter les épisodes d'étourdissement, l'hypokinésie, et les troubles crâniens.

7. Utilisation d'un composé selon l'une quelconque des revendications 1 à 3, dans la fabrication d'un médicament destiné à traiter les troubles neurodégénératifs.

8. Utilisation d'un composé selon l'une quelconque des revendications 1 à 3, dans la fabrication d'un médicament destiné à traiter la dépression.

9. Utilisation d'un composé selon l'une quelconque des revendications 1 à 3, dans la fabrication d'un médicament destiné à traiter l'anxiété.

10. Utilisation d'un composé selon l'une quelconque des revendications 1 à 3, dans la fabrication d'un médicament destiné à traiter la panique.

11. Utilisation d'un composé selon l'une quelconque des revendications 1 à 3, dans la fabrication d'un médicament destiné à traiter la douleur.

12. Utilisation d'un composé selon l'une quelconque des revendications 1 à 3, dans la fabrication d'un médicament destiné à traiter les troubles neuropathologiques.

13. Utilisation d'un composé selon l'une quelconque des revendications 1 à 3, dans la fabrication d'un médicament destiné à traiter les troubles du sommeil.

14. Composé selon l'une quelconque des revendications 1 à 3, destiné à être utilisé en médecine.
